# EUROPEAN PATENT APPLICATION

(11) **EP 1 138 781 A2**
(43) Date of publication of application: **04.10.2001**
(21) Application number: 01106806.1
(22) Date of filing: 19.03.2001
(51) Int. Cl.: C12Q 1/68, G01N 33/53

(54) **Method for Quantifying DNA binding activity of DNA binding proteins**

(30) Priority: 31.03.2000 US 539945
(71) Applicant: HEALTH RESEARCH, INC., Buffalo New York 14263 (US)
(72) Inventor: Kulesz-Martin, Molly F., Portland, Oregon 97219 (US); Liu, Yuangang, Portland, Oregon 97219 (US)
(74) Representative: Weber, Roland, Dipl.-Chem.

(57) **Abstract**

A method for quantifying a DNA binding protein in a biological sample. The method may be used in the absence of radioisotopes, and at a sensitivity greater than the commonly used electrophoretic mobility shift assay (EMSA) method previously discussed. In particular, the method includes the steps of: a) treating the sample to obtain a liquid to be tested for the DNA binding protein, b) incubating the liquid containing the DNA binding protein with a DNA, containing a binding sequence to which DNA binding protein can specifically bind so that DNA binding protein specifically binds to the DNA, c) separating the DNA with bound DNA binding protein from other DNA and proteins in the liquid, and e) quantifying the DNA binding protein without interference from other proteins of similar molecular weight in the absence of the use of a radioisotope, and at a sensitivity greater than EMSA. The amount of DNA binding protein may be quantified in several ways, e.g. directly by immunoreaction to the DNA binding protein or indirectly by amplification of DNA specifically bound to the binding protein followed by quantification of the amplified DNA. In a further embodiment of the invention, the total protein in the sample, having at least one specific immunoreactive site in common with the DNA binding protein, is quantified and compared with the quantity of binding protein to give an indication of relative proportions of bound and unbound protein in the total protein.

## Description

### Background of the Invention

This invention relates to quantifying DNA binding proteins and more particularly relates to quantifying DNA binding of DNA binding proteins.

The present invention especially relates to methods and kits applicable for the quantification of DNA binding activity of DNA binding proteins for use in both clinical and laboratory settings. Such quantifications may be used both for research and for reliable prognostic indicators in many diseases, e.g., cancer.

As a group, DNA binding proteins, i.e., proteins that, in their active forms, specifically bind to DNA, usually, but not essentially, in cell nuclear material, function through specific, direct interactions with DNA. They play pivotal roles in normal cellular activities as transcription factors that govern gene expression, steroid hormone receptors that confer specialized function, and DNA repair proteins that maintain the integrity of the genome. DNA binding proteins bind to DNA through non-covalent interactions such as hydrogen bonds, ionic bonds, and hydrophobic interactions. Although weak, the interactions between DNA and DNA binding proteins are among the most specific non-covalent interactions known in biology, due to the involvement of multiple contacts on a large interface between DNA and the binding protein. A subtle change of either DNA or protein may dramatically abolish the interaction; thus, delicate alterations in DNA binding proteins may result in dramatic changes in cellular activities. A number of oncogenes and tumor suppressor genes have been identified as DNA binding proteins, including myc, jun/fos, myb, erb/A, NFκB and p53. Other DNA binding proteins, for example, include p53 and estrogen receptor (ER). Although extensive studies have been carried out to elucidate their roles in tumorigenesis, the value of DNA binding proteins in clinical applications for tumor diagnosis and prognosis has not yet been fully realized.

p53 is one of the most frequently altered DNA binding proteins detected in human cancer, see e.g., Harris, C.C. and Hollstein, M. (1993), "Clinical Implications of the p53 Tumor-Suppressor Gene," New England Journal of Medicine, *329*, 1318-1327, incorporated herein by reference as background art. Wild type p53 mediates cell cycle checkpoints, allowing the repair of damaged DNA or triggering apoptosis in cells with irreparable DNA damage. A common mechanism of tumor response to various chemotherapeutic drugs has been attributed to p53-mediated apoptosis. However, use of p53 mutation as a reliable prognostic marker in the clinic is not as effective as desired. The current approaches used in the clinic may not be sensitive and specific enough to reflect the accurate status of p53 in cancer populations. Immunohistochemistry (IHC), single-strand conformation polymorphism (SSCP), and the yeast functional assay are current methods used to evaluate p53 status. IHC detects only the antigenicity of the protein and does not necessarily reflect the functionality of the detected protein. SSCP combined with DNA sequencing, although capable of pinpointing p53 mutations, needs additional information to distinguish silent mutations and functional mutations. A yeast-based functional assay has detected p53 mutations more precisely at a functional level; however, it has narrower applications since p53 can be inactivated without mutation. In addition to p53 mutations, p53 inactivation can occur due to viral oncogenes such as E6 protein of human papillomavirus; defects in the upstream pathways that induce and activate p53, such as ATM and DNA dependent protein kinase; and overexpression of mdm-2, which mediates ubiquitination of p53 for degradation. Therefore, a functional assay based not only on p53 genotype, but also on the integrity of the p53 pathway, should be more reliable for prognosis and treatment planning.

The molecular basis for p53 transactivation is binding to p53 consensus DNA sequences found in the regulatory region of its downstream genes. It might seem possible to detect the expression of the downstream genes (for instance, p21) as an indicator of p53 status in tumor cells. However, p21 expression can also be regulated by pathways independent of p53. Furthermore, p21 expression is associated with better prognoses in certain cases but worse prognoses in others. Because sequence-specific DNA binding is considered essential for p53 activities, it provides a means of functional assessment beyond the mere presence of the protein, since p53 protein can be present in cells yet be inactive. By measuring the DNA binding activity of p53 protein, it is possible to detect p53 abnormalities resulting from gene mutation as well as from post-translational modifications. The most commonly used approach for detecting DNA binding, the electrophoretic mobility shift assay (EMSA), has been widely used for analysis of DNA binding proteins, see e.g., Garner, M.M. and Revzin, A., (1981), "A Gel Electrophoresis Method for Quantifying the Binding of Proteins to Specific DNA Regions: Application to Components of the Escherichia Coli Lactose Operon Regulatory System," Nucleic Acids Research, *9*, 3047-3060, incorporated herein by reference as background art. Drawbacks of this assay restricting its application in the clinic include: (1) difficulty in interpretation and quantification of results, (2) poor sensitivity due to limited reaction volume, and (3) handling hazardous radioactive materials. For the purpose of enhancing sensitivity, the McKay assay has been developed in which protein/DNA complexes are enriched by immunoprecipitation with corresponding antibody, see e.g., McKay, R.D., (1981), "Binding of a Simian Virus 40 T Antigen-Related Protein to DNA," Journal of Molecular Biology, *145*, 471-488, incorporated by reference as background art. However, the quantification of DNA binding protein has to be done in separate aliquots by immunoblotting or ELISA, thereby restricting objective quantification. As a derivative of EMSA, the McKay assay uses radioisotopes, greatly limiting its general practice in the clinic. The analysis of p53 DNA binding for cancer prognosis calls for an alternative DNA binding assay that is more sensitive, quantifiable, and feasible for both clinical and laboratory use.

### Brief Description of the Invention

In accordance with the invention a method is provided for quantifying a DNA binding protein in a biological sample. The method may be used in the absence of radioisotopes, and at a sensitivity greater than the commonly used electrophoretic mobility shift assay (EMSA) method previously discussed. In particular, the method includes the steps of:
a) treating the sample to obtain a liquid to be tested for the DNA binding protein,
b) incubating the liquid containing the DNA binding protein with a DNA, containing a binding sequence to which DNA binding protein can specifically bind so that DNA binding protein specifically binds to the DNA,
c) separating the DNA with bound DNA binding protein from other DNA and proteins in the liquid, and
d) detecting the separated DNA binding protein, in the absence of the use of a radioisotope, and at a sensitivity greater than EMSA, using a test that yields a first numerical result proportional to the quantity of separated DNA binding protein, said test being selected from the group consisting of immunological quantification of DNA binding protein separated with the bound DNA and PCR amplification of DNA separated with the DNA binding protein followed by quantification of the PCR amplified DNA. The method further includes the embodiment where total protein in the sample, having a specific immunoreactive site in common with a specific immunoreactive site of the separated DNA binding protein, is detected, using a test that yields a second numerical result proportional to the quantity of said total protein. The first numerical result may then be compared with the second numerical result to obtain an index reflecting relative proportion of bound and unbound protein in said total protein.

In practicing the method, the desired DNA with attached binding protein may be separated from other DNA's and proteins in the liquid in several ways.

The DNA, containing a binding sequence to which DNA binding protein can specifically bind, may be treated with a material to provide a site that permits the treated DNA to be separated from other DNA in the liquid, without interfering with the binding sequence, before the liquid containing the DNA binding protein is incubated with the treated DNA so that DNA binding protein specifically binds to the DNA. The treated DNA with bound DNA binding protein may then be separated from other DNA in the liquid using the provided separation site. As an example of such a method, the DNA may be biotinylated and removed by reaction with a captive agent, e.g. streptavidin, secured to a substrate, e.g. magnetic beads.

As another example, the DNA, prior to being reacted with DNA binding protein may be treated by providing a restriction site in all DNA in the liquid, which restriction site can be protected, by bound binding protein, from destruction by a restriction enzyme specific for the restriction site. DNA bound to binding protein may then be separated from other DNA in the liquid by exposing DNA in the liquid to the restriction enzyme thus destroying non-bound DNA.

After being separated from other DNA and proteins in the liquid, the amount of DNA binding protein may be quantified in several ways. In a first general method for quantification, the binding protein is directly quantified, e.g. by being freed from the DNA after removal of the DNA, and being subjected to SDS-gel fractionation followed by quantification by immunoblotting using an antibody specific for the particular DNA binding protein. For example, where the binding protein is p53, it may be immunoblotted using p53 antibody.

In a second general method, the DNA binding protein is indirectly quantified by quantifying the DNA to which it was bound, e.g., by subjecting the DNA removed with bound DNA binding protein to polymerase chain reaction (PCR) amplification. The amplified DNA may be quantified by a number of methods, e.g. by a detection method selected from agarose gel electrophoresis with DNA staining, enzyme linked oligonucleotide sorbent assay (ELOSA) and PCR with beacon oligonucleotide as primers by spectrofluorometric thermo cycler.

The total protein, in the sample, that has the common specific immunoreactive site with the active DNA binding protein, is usually quantified by an immunoassay, e.g. immunoblotting, using an antibody specific for the immunoreactive sequence (DNA affinity immunoblotting (DAI).

In another embodiment for determining total protein, the total protein is drawn from the sample by an antibody to the immunoreactive site. A DNA treated to bind to the total protein is bound to the total protein. The bound DNA is then amplified by PCR and quantified to give a numerical result proportional to the total protein.

More particularly, as an example, the total protein in the sample, having at least one specific immunoreactive site in common with the DNA binding protein, may be quantified by subjecting a sample of the liquid containing the total protein to SDS-gel fractionation followed by quantification by immunoblotting, in parallel with immunoblotting to determine binding protein, using a same antibody reactive with the immunoreactive sequence and comparing the quantity of the total protein with the quantity of binding protein.

An embodiment of a general method for directly quantifying DNA binding of a DNA binding protein in a biological sample and comparing it with total protein in the sample having at least one specific immunoreactive site in common with the DNA binding protein, includes the steps of:
a) treating the sample to obtain a liquid containing the total protein including the DNA binding protein,
b) generating a DNA template, containing a binding site to which DNA binding protein can specifically bind, with a material to provide a site that permits the DNA to be removed from solution without interfering with the binding sequence,
c) incubating the liquid containing the DNA binding protein with the DNA template so that DNA binding protein specifically binds to the DNA to form a complex,
d) removing the DNA template with bound DNA binding protein from the liquid using the provided removal site,
e) separating the bound DNA binding protein from the DNA,
f) quantifying the DNA bound protein,
g) quantifying the total protein in the liquid, and
f) comparing the quantity of DNA binding protein with the total protein to obtain an index reflecting relative proportions of bound and unbound protein in the total protein.

Since most DNA binding proteins are found with DNA located within the nucleus of the cell, the DNA binding proteins are commonly nuclear proteins and the samples to be tested usually, but not always, contain nuclear materials. Although other biological samples may be used, especially those that may contain excreted nuclear waste products, e.g. serum or ascites, the most common source of samples to be tested are cellular lysates such as tumor cell lysates. protein.

In accordance with the invention, a plurality of different DNA binding proteins having different molecular weights may be simultaneously quantified using a plurality of DNAs, which contain binding sites to which the different DNA binding proteins can specifically bind. In such a case, all of the DNAs containing the specific binding sequences may be treated with a material to provide a site that permits the treated DNAs to be separated from other DNA in the liquid without interfering with the binding sequences. The binding proteins should have different molecular weights so that they do not interfere with each other during detection and quantification. treated DNAs to be separated from other DNA in the liquid without interfering with the binding sequences.

DNA affinity immunoblotting as previously discussed may be used for research or therapeutic purposes in: (1) detecting active DNA binding proteins relevant to a particular disease; (2) isolating and identifying DNA binding proteins and their associated proteins; and (3) screening compounds that target DNA/protein interactions. The highly sensitive DNA affinity PCR may be used in detecting trace amounts of DNA binding proteins of clinical significance, e.g., in molecular diagnostics, as intermediate endpoints in prevention and therapeutic clinical trials, and for molecular profiling of individual patient tumors. Molecular profiling can assist patients and their physicians in decisions about treatments. The test will further be useful for future individualized therapies targeted to molecular defects driving a given patient's disease.

### Brief Description of the Drawings

Figure 1 is a flow diagram illustrating a method for quantification of total p53 protein and bound p53 protein by parallel immunoblotting.

Figure 2 is a comparison chart showing sensitivity for quantification of p53 protein active in DNA binding, competition for sequence specific DNA binding and measurement of functional p53 proteins in cells with different p53 genotypes.

Figure 3 illustrates functional analysis of p53 and ER proteins in a mouse mammary model using immunoblotting.

Figure 4 illustrates identification of p53 binding to mismatched DNA structure by DNA affinity immunoblotting.

Figure 5 is a schematic diagram of DNA affinity assays using PCR for separately performed active binding protein and total protein.

Figure 6 is a schematic diagram of DNA affinity assays using PCR for parallel trapping and PCR amplification of binding and non-binding p53 and ER proteins.

Figure 7 illustrates a microtiter plate with bound PCR amplified DNA visualized by ethidium bromide staining.

Figure 8 illustrates DNA binding activities of p53 and ER proteins in human breast cancer by DAI.

### Detailed Description of the Invention

An objective of the present patent application is therefore to provide a method for determining quantitatively the DNA binding activities of DNA binding proteins.

Unless otherwise indicated in the text of the specification, the following terms and abbreviations have the indicated associated meanings.

"291 cells" are cells from a well known and readily available non-transformed mouse epidermal cell line.

"3C3 DNA" is a mismatched DNA template containing unpaired cytosines.

"2C3C DNA" is a control DNA for 3C3 DNA that does not contain mismatched cytosines.

"Agarose gel electrophoresis" a well known method for separating DNA by size by electrophoresis through agarose gel.

"Apoptosis is programmed cell death.

"ATM" is an ataxia telangiactasia mutation

"Biotin molecule" is molecule with MW 244 that binds to avidin, streptavidin specifically.

"Biotinylate" means to modify by covalent conjugation with one or more biotin molecules.

"Blocking DNA" is a non-specific DNA that is used to enhance the specificity of specific DNA binding by occupying non-specific sites in a DNA binding reaction.

"BSA" is bovine serum albumin.

"Cell line 480" is a well known and readily available human adenocarcinoma cell line.

"CL-4B" is Sepharose CL-4B, a commercially available inert chromatographic support matrix.

"DAI" means DNA affinity immunoblotting of the invention.

"DAP" means DNA affinity polymerase chain reaction.

"DNA binding protein" means a DNA active form of a protein, i.e. a protein that specifically binds to a specific site on a DNA molecule and as such affects the function of the DNA. As used in the claims "DNA binding protein" refers to a protein that is in active binding form as opposed to a form that does not specifically bind to the specific site on the DNA. As is apparent from context, within the specification, "DNA binding protein" may be generically used to mean a specific type of protein in both its DNA binding (active) and non-binding (inactive) forms, i.e. collectively "total protein" below.

"DNA template" as used in the claims is a form of DNA specifically selected or designed to have a specific binding site for a particular DNA binding protein.

"DO1" is a polyclonal antibody specific for p53 protein.

"DTT" is dithioerythreitol, a reducing agent that maintains -SH groups in a reduced state.

"ELISA" is enzyme-linked immunosorbent assay.

"ELOSA" is enzyme-linked oligonucleotide sorbent assay.

"EMSA" is electrophoretic mobility shift assay.

"Epitope" is a portion of a protein that is recognized by an antibody.

"ER" is estrogen-receptor.

"ERE" is estrogen response element.

"Estradiol" is a steroid produced by the ovaries.

"Fluorescein" is a fluorescent substance here used as a hapten label in oligonucleotide.

"Fluorophore" is a substance that generates fluorescence.

"FNA" is fine needle aspirate.

"Hapten-labeled oligonucleotide" is an oligonucleotide labeled with small substance recognizable by specific antibody, e.g. fluorescein, digoxigenin.

"Holliday juncture" is an intermediate DNA structure in DNA recombination.

"IHC" means Immunohistochemistry.

"ImageQuant" is a software program designed for the analysis of electrophoretic assays.

"Immunoprecipitation" is the isolation of protein using specific antibodies.

"kD" is kilodalton (unit of measure).

"Magnetic streptavidin" means magnetic beads cross-linked with streptavidin.

"Microsequencing" is a method for determining the sequence of DNA or proteins.

"Oligomerization" is the ability of proteins to join in different conformations.

"Oligonucleotide" is a compound of a small number of nucleotide units.

"PAB 122" is an antibody specific for p53 protein.

"PAb421" is an antibody specific for p53 protein.

"Palindromic DNA" is a DNA segment that can be read identically from both 5' and 3' directions.

"PCR" means polymerase chain reaction.

"Poly(dI-dC)" are random DNA sequences (for a control).

"PR" means progesterone receptor.

"SDS-PAGE" is sodium dodecyl sulfate-polyacrylamide gel electrophoresis (a method).

"SSCP" means single-strand conformational polymorphism.

"Surface Enhanced Laser Desorption/Ionization Mass Spectrometry" is a well known method for determining the weight of proteins.

"SV40 large T antigen" is a viral protein from SV40 virus that specifically binds to p53 protein.

"SW837" is a well known and readily available human adenocarcinoma cell line.

"TATA" is a nucleotide sequence (Thymine and Adenine) that controls general transcription.

"TBP" is TATA binding protein.

"TM cells" are murine tumor mammary cells.

"Total protein" means all of a specific type of protein having a specific immunoreactive site in common with a specific active DNA binding protein. Total protein includes both active and inactive forms of the specific type of protein. Proteins within "total protein" usually have significant lengths of amino acids having the same sequence, e.g. usually over 80% and often over 95% sequence identity.

"TRITON X-100" is a non-ionic surfactant.

In a first embodiment, the invention provides a method for measuring the ratio of active protein that binds to DNA and the total protein in tissue lysate from surgical tumor tissue specimens or cells. The detection of active DNA binding proteins may be achieved by the enrichment of DNA bound protein using biotinylated DNA and magnetic streptavidin. Fractionation of DNA bound proteins and total proteins through SDS-PAGE, followed by immunoblotting with corresponding antibodies, makes it possible to measure DNA bound and total forms of multiple DNA binding proteins simultaneously (provided that they are different in molecular weight). Since DNA bound proteins and total proteins are detected under identical conditions, it allows objective quantification of relative DNA binding activity and expression as a functional index. This functional index is more accurate in reflecting functional status of DNA binding proteins than detecting absolute DNA binding by EMSA or other conventional DNA binding assays. The utility of this assay can be extended to identify proteins that bind to DNA with unique structures. Further, this test can detect DNA binding proteins at levels of less than 100 µg without employing radioactive isotopes.

In a second embodiment, the invention provides a highly sensitive DNA binding assay capable of detecting DNA binding activities with micro-samples from core needle biopsy or other minute clinical specimens. PCR is integrated in this assay to amplify protein-bound DNA as a readout for DNA binding activity of the tested DNA binding protein.

The test is so sensitive that only a few hundred molecules of a particular binding protein may be detected. The method described may for example be used as a PCR-based detection system for p53 and estrogen receptor (ER) quantification using a bifunctional fusion protein of SV40 T antigen and streptavidin and a biotinylated estradiol, respectively. The quantification of p53 and ER by this PCR-based detection system provides not only a denominator for calculation of functional index of the DNA binding protein, but also a reference for evaluating other biochemical features of the DNA binding proteins, such as conformation for p53 or ligand binding for ER.

The methods of the present invention provide simple, non-radioactive assays that can be used to detect and quantify the functional status of DNA binding proteins that are significant in human disease, by reflecting severity, prognosis, and integrity of the cellular response to treatments. The implementation of these assays may be useful for physicians and patients in making decisions about therapeutic regimens and monitoring therapeutic response during treatment, for example in cancer therapy. Functional status of p53, ER and other DNA binding proteins should be more powerful in predicting patient prognosis, survival, and response to available treatments than immunohistochemistry or other current assessments of protein expression alone. As a database of patient tumor protein functional profiles develops through implementation of these assays, patients and medical caregivers may use it to make more informed decisions among available standard treatments, or to elect more aggressive experimental treatments or no treatment based on evidence of benefit.

The methods of the invention may be illustrated by following discussions related to the DNA binding proteins p53 and estrogen receptor protein (ER). The references referred to herein are incorporated by reference as background art.

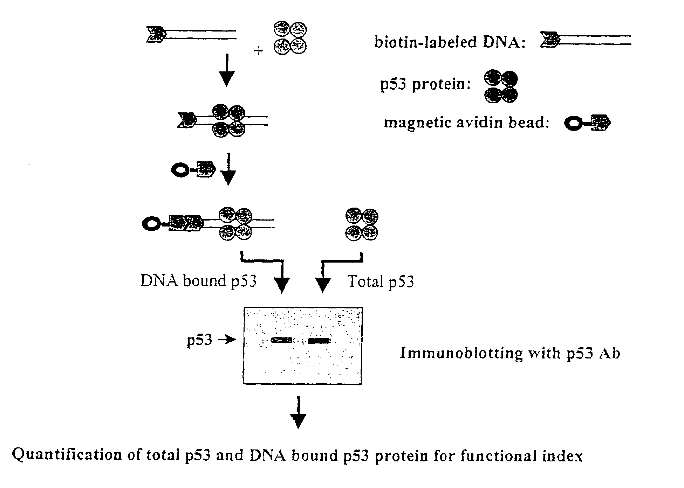

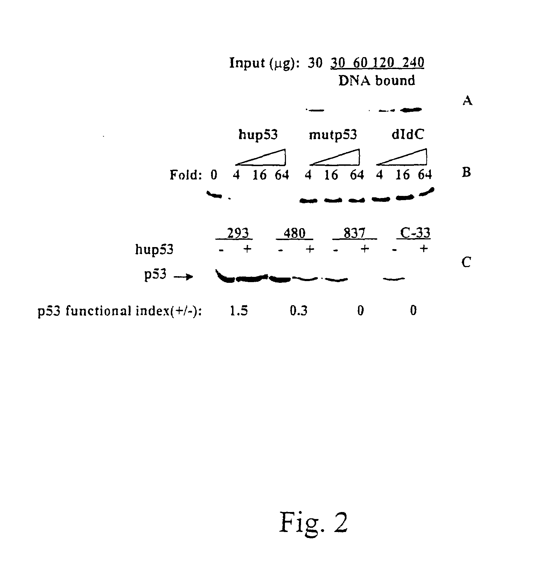

An embodiment of the invention, the DNA affinity immunoblotting (DAI) assay is directed toward the detection of specific DNA/protein interactions at a level of sensitivity beyond that reproducibly detectable using standard DNA binding assays like EMSA. The novel features of the DAI are: 1) ability to quantify the functional fraction of total particular DNA binding protein in the sample; 2) the application of this functional assessment to tumor and cell samples for clinical and research benefit; 3) the ability to functionally assess multiple DNA binding proteins in the same sample; and 4) the modifications detailed below which make the methods specific, quantifiable and applicable to tissue and cell samples relevant to human disease. The DAI method combines and modifies two common techniques, biotin/streptavidin affinity chromatography (Kadonaga J.T. and Tjian R. (1986), "Affinity purification of sequence-specific DNA binding proteins", Proceedings of the National Academy of Sciences of the United States of America *83*, 5889-5893) and immunoblotting (Burnette W.N. (1981), "Western blotting: electrophoretic transfer of proteins from sodium dodecyl sulfate―polyacrylamide gels to unmodified nitrocellulose and radiographic detection with antibody and radioiodinated protein A", Analytical Biochemistry *112*, 195-203). The strategy is to enrich endogenous DNA binding proteins in a specific complex with biotinylated target DNA molecules by incubation with streptavidin, then to detect the particular proteins specifically by immunoblotting (Fig 1). More specifically, Figure 1 illustrates quantification of total p53 and DNA bound p53 protein for use in a functional index. Figure 1 is a schematic illustration of DNA affinity immunoblotting. In a DNA binding reaction, p53 that is active in DNA binding will bind to a biotinylated DNA duplex with p53 consensus sequence. The p53-biotinylated DNA complex can be captured by magnetic streptavidin and subjected to SDS-gel fractionation followed by immunoblotting with p53 antibody. The relative DNA binding activity of p53 can be calculated by the signal intensity of DNA bound p53 to that of total p53 as functional index. Experiments performed in support of the present invention measured DNA binding of p53 protein to a consensus p53 binding sequence containing a palindromic DNA motif with two repeats of PuPuPuCATGPyPyPy (SEQ. I.D. #1) (el Deiry W.S., Tokino T., Velculescu V.E., Levy D.B., Parsons R., Trent J.M., Lin D., Mercer W.E., Kinzler K.W., and Vogelstein B., (1993), WAF1, a potential mediator of p53 tumor suppression, Cell 75, 817-825). The 5' end of one strand of the DNA duplex containing the p53 consensus sequence is biotinylated and annealed with its complementary strand which is not biotinylated. DNA binding reactions are performed in DNA binding solution containing 200 µg of high-salt extracts of tumor tissue samples or cultured tumor cells, 10 pmol biotinylated p53 DNA duplex, 2 µg of poly(dI-dC), and 0.1 mg of magnetic streptavidin beads, in 400 µl of DNA binding buffer (20 mM Tris-HCl [pH 7.2], 80 mM NaCl, 1 mM EDTA, 0.1% Triton X-100, 4% glycerol, 5 mM DTT). The DNA/protein complexes are pelleted using a magnet and washed three times with the DNA binding buffer. The bound proteins are analyzed alone with 40 µg of corresponding high salt extracts by polyacrylamide/SDS gels followed by immunoblotting with antibody specific to p53. A key element for DAI is that there is no limitation in reaction volume, allowing the desired sensitivity to be achieved by increasing the amount of cell lysate in the DNA binding reaction. EMSA is limited by reaction volume, i.e. the size of the well in a non-denaturing gel, and by the achievable protein concentration from tissue samples. By increasing the volume of nuclear extract to fourfold the amount allowable for EMSA, we were able to detect DNA binding activity of p53 protein in normal epidermal 291 cells in the absence of PAb421 as activator within the reactions (Fig. 2A). The signal intensity of DAI with 120 µg nuclear extract is comparable to that of direct immunoblotting with 30 µg nuclear extract, indicating that a quarter of the total p53 protein detectable in the untreated cells is active in DNA binding. The advantages over previous assays are 1) sensitivity for untreated normal cells known to have low endogenous levels of active p53; and 2) the ability to detect activity of p53 without the use of PAb421 p53 antibody as an artificial stabilizer in the *in vitro* reaction.

Employing two specific agents in distinct steps ensures specificity of the DAI assay. The first agent, in the case of p53, may be a biotinylated p53-specific consensus DNA fragment that captures p53 protein capable of DNA binding. The second agent, in the case of p53, is p53 specific antibody for detecting DNA-bound p53 by immunoblotting. As shown in Figure 2B, DNA binding by p53 proteins was specific to the biotinylated p53 consensus sequence but not to a mutated control sequence. The p53 signal was abrogated by an unbiotinylated p53 consensus sequence but not by poly(dI-dC) or mutant p53 DNA as competitors. The specificity of DAI was established further by using nuclear extracts from cells with different p53 genotypes (Fig. 2C). The DAI reactions (representing active p53 protein) were carried out on aliquots of nuclear extract and shown adjacent to direct immunoblotting of one fifth the aliquot (representing total p53 protein). These values were used to calculate a functional index convenient for comparison among samples [functional index = signal intensity of DNA bound p53 divided by signal intensity of total p53]. The true proportion of functional p53 protein in a cell sample is estimated by dividing the functional index by 5 in this case (the difference between the input of 40 µg and 200 µg lysate proteins for direct immunoblotting vs. DAI, respectively, chosen to give signal intensities within the linear range for both types of reactions). Significant DNA binding activity was detected in human 293 cells, consistent with their wild type p53 genotype. The p53 active for DNA binding was approximately one third of the total p53 protein (calculated by dividing the functional index by 5). p53 proteins from human adenocarcinoma cell line SW837 and cervical carcinoma cell line C-33 did not bind DNA, which is consistent with their mutant genotypes (248 Arg>Trp in SW837; 273 Arg>Cys in C-33). However, DNA binding activity was detected in human adenocarcinoma cell line 480 with the p53 273 Arg>His mutation. It is interesting to note that two different p53 mutations within the same codon (273) resulted in dramatic differences in p53 DNA binding activity. Active DNA binding of 273 Arg>His mutant has been observed by means of EMSA (Park D.J., Nakamura H., Chumakov A.M., Said J.W., Miller C.W., Chen D.L., and Koeffler HP, (1994), "Transactivational and DNA binding abilities of endogenous p53 in p53 mutant cell lines", Oncogene 9, 1899-1906). However, the differences in DNA binding activities of wild type p53 and its mutants can be measured more precisely and compared using DAI. Although DNA binding activity of p53 is obvious in 480 cells, it is significantly weaker than wild type p53 as indicated by the functional index (0.3 and 1.5, respectively).

In summary, Figure 2 illustrates DNA binding activities of p53 by DAI. 2A shows quantification of p53 protein active in DNA binding by DAI. Indicated amounts of lysate from 291 cells were subjected to DAI with PAb122. Thirty µg aliquot of lysate was loaded as reference for total p53 protein. 2B shows competition for sequence-specific p53 DNA binding. Aliquots of 200 µg of 291 lysate were subjected to DAI with indicated competitors. 2C shows measurement of functional p53 proteins in cells with different p53 genotypes. 200 µg of nuclear extract from each of the cell lines (see text) was subjected to DAI (+) and compared with total proteins (-).

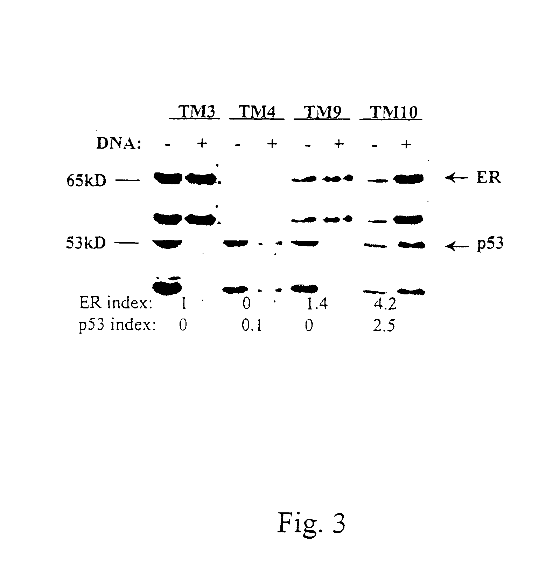

These studies were extended to p53 wild type and mutant proteins in mammary cells, using the mouse mammary cell model developed by Dan Medina (Medina D., Kittrell F.S., Liu Y.J., and Schwartz M. (1993a), "Morphological and functional properties of TM preneoplastic mammary outgrowths", Cancer Research *53,* 663-667). In addition, the studies offered the opportunity to compare the sequence-specific DNA binding detectable by DAI with previous EMSA results and to determine whether the DAI results predicted the transcriptional activity of p53 (Medina D., Kittrell F.S., Oborn D.J., and Schwartz M. (1993b), "Growth factor dependency and gene expression in preneoplastic mouse mammary epithelial cells", Cancer Research *53*, 668-674). Previous studies have shown that the p53 gene is wild type in weakly tumorigenic cell lines TM9 and 10 and mutant in a weakly tumorigenic line (TM13) and a highly tumorigenic line (TM4). The functional status of p53 in this mammary cell model was examined by DAI and the results are shown in Figure 3. p53 proteins from TM3 and TM4 did not bind DNA, or bound at negligible levels, which is consistent with their mutant genotypes. DNA binding activity is consistent with a wild type p53 genotype in the case of TM10 cells. However, p53 protein from TM9, which also contains only wild type p53, failed to bind DNA. Thus, DAI distinguished subsets of mammary tumors with wild type p53 genotype: tumors with wild type but non-functional p53 and tumors with functional wild type p53. This is consistent with evidence that p53 is inactivated by defects other than p53 mutation. Furthermore, the DAI results mirrored those of EMSA in these cells, yet had the advantage of providing total protein controls and more quantitative information for comparison among samples. Of significance to DAI as an indicator of function, DAI predicted p21 induction by DNA damaging agents in parallel experiments using these same cells.

An additional, unique advantage of DAI is that it can measure multiple DNA binding proteins simultaneously if they are of different molecular weight. This is particularly significant in breast cancer, ovarian cancer, and prostate cancer in which other DNA binding proteins have been identified as prognostic factors, such as steroid receptors for estrogen, progesterone, and androgen. Therefore, we examined the DNA binding activity of ER along with p53 in TM cells (Fig. 3). Biotinylated p53 consensus DNA and estrogen receptor element DNA were added in the same reaction. Since p53 (53kD) and ER (56kD) can be separated clearly by SDS-polyacrylamide gel electrophoresis, DNA bound proteins were analyzed by immunoblotting with antibodies for p53 and ER on the same blot. Of particular interest, not all the samples with immunodetectable ER showed corresponding DNA binding activities. This suggests that by distinguishing function from protein content, DAI has the potential to add additional information valuable for prognosis. Furthermore, the DAI assay has the potential to detect additional DNA binding proteins with different molecular weights; one example includes the 94 kD progesterone receptor. The ability to measure multiple factors in a single reaction makes it possible to develop DAI as a thorough assay for multiparameter analysis, molecular diagnostics, and treatment plan.

Another aspect of the invention establishes to tissue quality control to normalize DNA binding activity affected by the quality of tumor tissue. General transcription factors are selected as internal controls for tissue quality. One example is the TATA binding protein (TBP), a general transcription factor that recognizes TATAAAA (SEQ. I.D. #2) and closely related sequences, referred to as TATA boxes. The molecular weight of TBP is 36 Kd, permitting it to be distinguished from p53 (53Kd) and ER (65Kd). Biotinylated DNA with the TBP binding site TATAAAA and its flanking sequence is reacted in solution containing tissue lysate and biotinylated p53 DNA duplex. The DNA/protein complexes containing TBP and p53 protein are captured by magnetic streptavidin beads as described above. The DNA/protein complexes are fractionated by SDS-PAGE, followed by immunoblotting with antibodies for TBP and p53. The lysate quality, as indicated by TBP functional index, is calculated by dividing the signal intensity for DNA bound TBP by that for the total TBP. This value can be used to standardize the functional indices generated for p53 and other DNA binding proteins in the same reactions. The absolute functional index for p53 is calculated by dividing the signal intensity for DNA bound p53 by that for the total p53 protein. The relative functional index of p53 is determined by dividing with the absolute functional index of TBP (functional index of p53= [signal intensity of DNA bound p53/ signal intensity of total p53]÷[signal intensity of DNA bound TBP/ signal intensity of total TBP]).

In summary, Figure 3 shows functional analysis of p53 and ER in the mouse mammary model. 200 µg of nuclear extracts from each of the cell lines were reacted with a biotinylated DNA cocktail containing p53 consensus sequence and ERE sequence. The gel was blotted with ER antibody (HC-20) and p53 antibody (122). The bands were quantitated using ImageQuant. The functional index was calculated by dividing signal intensity of DNA bound protein (+) by signal intensity of 40 µg of total protein (-).

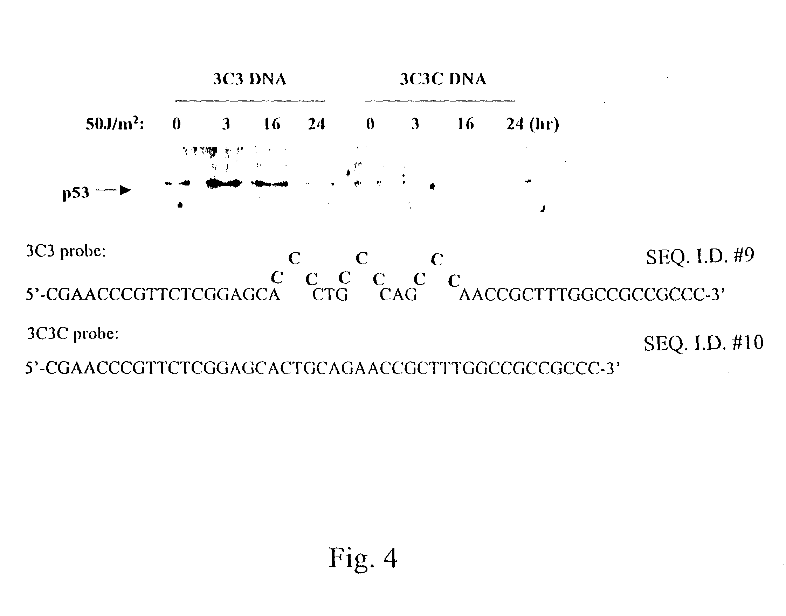

In addition to using biotinylated DNA duplex in analysis of DNA binding proteins, the utility of DAI can be extended to identify proteins that bind to unique DNA structures by using, for example, biotinylated DNA with Holliday juncture, an intermediate DNA structure in DNA recombination (Holliday R, (1974), "Molecular aspects of genetic exchange and gene conversion", Genetics *78*, 273-287), or DNA with mismatch structure (Fig. 4). The bound proteins can be detected by specific antibodies if the bound proteins are known. Alternatively, the bound proteins can be analyzed by microsequencing gel fractionated polypeptides (Albor A., Kaku S., and Kulesz-Martin, M. (1998), "Wild-type and mutant forms of p53 activate human topoisomerase I: a possible mechanism for gain of function in mutants", Cancer Research *58*, 2091-2094) or in combination with protein chip analysis (Kuwata H., Yip T.T., Yip C.L., Tomita M., and Hutchens T.W., (1998), "Bactericidal domain of lactoferrin: detection, quantitation, and characterization of lactoferricin in serum by SELDI affinity mass spectrometry", Biochemical & Biophysical Research Communications *245*, 764-773). The bound proteins can be eluted from streptavidin/biotinylated DNA complex by digestion with either DNase or restriction enzymes (restriction sites can be introduced into the biotinylated DNA for this purpose). The DNAse and restriction enzymes can be absorbed by heparin-agarose beads after the digestion. The eluted proteins are suitable for SDS-PAGE, and proteins of interest can be excised for microsequencing. Alternatively, the eluted proteins can be subjected to protein chip analysis by Surface Enhanced Laser Desorption-Ionization Mass Spectrometry.

In summary, Figure 4 shows identification of p53 binding to mismatched DNA structure by DNA affinity immunoblotting. Nuclear extracts were prepared from 291 cells at indicated time points after 50J/m² UV-irradiation. Binding to mismatched DNA was analyzed with 200 µg of nuclear extracts in reactions with 0.5 µg biotinylated mismatched DNA(3C3), and its control DNA 3C3C probe. The DNA-protein complexes were captured by magnetic streptavidin beads and subjected to 1% SDS-PAGE followed by blotting with PAb122.

Current assays for screening compounds that target DNA-protein interactions are mainly based on *in vitro* DNA binding assays, e.g., EMSA using recombinant protein and radiolabeled DNA. For developing a compound to be a clinically applicable drug, the function of the compound should be evaluated not only at a molecular level but also at a cellular, and more importantly, a physiological level. Due to the sensitivity and ability to quantify DNA binding endpoints afforded by the current assay, compound screening for ability to target DNA-protein interactions can be done not only at the molecular level currently practical, but also at the cellular and functional -physiological levels. The sensitivity and specificity of DAI makes it possible to evaluate the function of compounds in complex cellular physiological contexts by using cell or tissue lysates in the DNA binding assay in the presence of test agent. More importantly, it permits treatment of the animal and evaluation of activities in target tissues, by harvest of the tissues for DAI and quantification of effects on specific DNA binding proteins therein.

A second embodiment of the present invention is DNA affinity PCR. As cancer is being diagnosed earlier, the size of surgical pathology specimens is diminishing. In addition, the smaller surgical specimens are under increasing demand for diagnostic assays. Therefore, new assays sensitive enough for micro-samples would provide a distinct advantage for translating basic knowledge about cancer genes to practical applications in the clinic. Moreover, clinical assays are challenged further where sampling of smaller tumors or of high risk populations on prevention trials means that specimens from core biopsy or fine needle aspirates (FNA) may be the only available specimens. Functional assessment of p53 and other DNA binding proteins in micro-samples from FNA and core biopsy may provide information not only for diagnosis and therapy but possibly also for chemoprevention. One drawback of DAI is that it requires minimally 100 mg of tumor tissue (0.3 mm³). This is achievable in many surgical specimens but is beyond the scale of samples from core needle biopsy or FNA. Enhanced sensitivity of DAI is at least partly based on increased sample loading, leaving no room for DAI to be developed for micro-tumor samples.

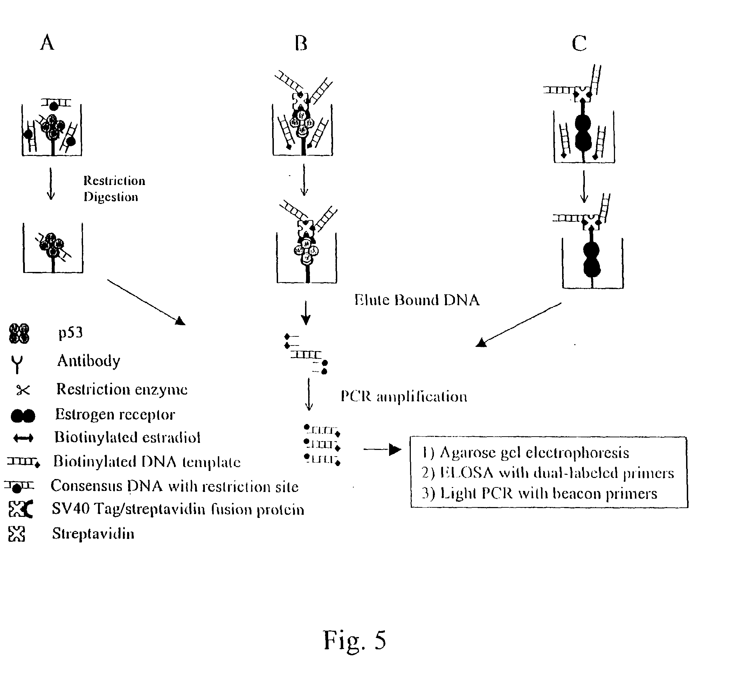

The second embodiment of the invention presents a more sensitive assay, DNA affinity PCR (DAP), making it possible to detect DNA binding activities of p53 and other DNA binding proteins in micro-tumor samples. The strategy of DNA affinity PCR (DAP) is to capture specific protein-DNA complexes on a solid matrix, followed by PCR amplification of the bound DNA fragment, e.g. as shown in Figure 5. More specifically, Figure 5 shows a schematic illustration of DNA affinity assays. 5A shows DNA affinity PCR where functional DNA binding proteins in the lysate will bind to DNA template with consensus sequence and restriction sites. The bound DNA will be captured by antibody immobilized in microplates whereas the unbound DNA template will be digested by restriction enzymes. The signal of bound DNA will be amplified by PCR. 5B shows modified immuno-PCR for p53 protein quantification. p53 protein in lysate will form a complex with SV40 Tag/streptavidin fusion protein and biotinylated DNA template. The complex will be captured by anti-p53 antibody immobilized in microplates. The signal of bound DNA will be amplified by PCR. 5C shows modified immuno-PCR for ER quantification. ER protein that is active for binding estradiol will form a complex with biotinylated estradiol and streptavidinbiotinylated DNA template. The complex will be captured by anti-ER antibody immobilized in microplates. The signal of bound DNA will be amplified by PCR. The amplified DNA can be detected and quantified by 1) agarose gel electrophoresis; 2) enzyme-linked oligonucleotide sorbent assay (ELOSA) with one primer labeled with biotin and the other with fluorescein; or 3) PCR with beacon oligonucleotide as primers by a spectrofluorometric thermal cycler. The major advantage of DAP is high sensitivity due to the bound DNA being amplified by PCR, the most powerful amplification system currently available (Saiki R.K., Scharf S., Faloona F., Mullis K.B., Horn G.T., Erlich H.A., and Arnheim N. (1985), "Enzymatic amplification of beta-globin genomic sequences and restriction site analysis for diagnosis of sickle cell anemia", Science *230*, 1350-1354). Under general reaction conditions, a million fold amplification can be easily achieved. Theoretically, in combination with enzyme-linked oligonucleotide sorbent assay (ELOSA) (Hahn M., Dorsam V., Friedhoff P., Fritz A., and Pingoud A., (1995), "Quantitative polymerase chain reaction with enzyme-linked immunosorbent assay detection of selectively digested amplified sample and control DNA", Analytical Biochemistry *229*, 236-248), DNA affinity PCR could be a million times more sensitive than standard ELISA. The ELOSA is capable of detecting 0.1-1 fmol of a specific DNA sequence. Thus, the DNA affinity assay, combined with the use of ELOSA, could conceivably permit quantification of a few DNA binding events per sample, i.e. in a few tumor cells. However, non-specific DNA binding could be the major obstacle to the sensitivity of DAP. Powerful PCR would amplify both specifically-bound DNA and non-specifically bound DNA. Non-specific DNA binding may occur among the solid matrix, immobilized antibodies, and non-specific proteins. Due to the sensitivity of DAP, a few non-specifically bound DNA's might also be detected. Therefore, the sensitivity of DNA affinity requires that specific DNA binding events greatly outnumber nonspecific DNA binding in order to reach a significant difference between positive and negative samples. Therefore, decreasing non-specific DNA binding is critical for the sensitivity of the DNA affinity PCR. To reduce non-specific binding, a blocking DNA may be used that is designed to be identical to the specific template DNA except for mutated binding sites and lack of primer sequences for the template DNA. For example, in the case of p53 and estradiol, a mutant p53 or estrogen response element (ERE) DNA fragment without flanking primer sequences may be used as blocking DNA instead of salmon sperm DNA. Consequently, the blocking DNA cannot be recognized by p53 and ER proteins or be amplified by PCR with template primers. Secondly, restriction sites may be introduced into the spacer region, flanking regions or both regions of the specific binding sites for each DNA binding protein. Sequence-specific binding will protect the restriction sites from digestion by the corresponding restriction enzymes. Unbound DNA can be digested and thus become inaccessible as a template for subsequent PCR amplification. Reduction of non-specific binding by orders of magnitude is expected by combining several restriction digestions and blocking with specific DNA. For example, three restriction sites may be introduced in the spacer region and 5' and 3' immediate flanking regions, respectively, so that the introduction of these restriction enzymes does not interfere with specific DNA binding. The integration of three restriction sites permits three options or combinations for restriction digestion to eliminate non-specific DNA binding in the DNA binding assay.

An example of this DNA template and blocking DNA is a 200 bp DNA template with a p53 or ER binding site constructed by cloning the binding site into a plasmid vector. The template is synthesized by PCR with a pair of primers that flank the binding site. The p53 consensus sequence (AGGCATGTCTgaattcAGGCATGTCT) (SEQ. I.D. # 3) with EcoRI restriction site introduced into the middle spacer and XhoI at the 5 end and BamHI at the 3 end respectively is synthesized. The integration of three restriction sites permit three options or combinations for restriction digestion to eliminate non-specific DNA binding in the DNA binding assay. The duplex DNA is prepared by mixing equal amounts of oligo DNA in annealing buffer (150 mM NaCl, 10 mM Tris HCl[pH 8.0], 5mM EDTA) and heating at 95°C for 5 min then cooling down to 60°C for 30 min. The annealed DNA is cloned into pBluescript, digested with XhoI and BamHI. The 5 template primer sequence (TTAAGTTGGGTAACGCCAGG) (SEQ. I.D. #4) and the 3 primer template (AAAGGGAACAAAAGCTGGGT) (SEQ. I.D. #5) that flank the cloned p53 binding site is synthesized for template amplification. A 210 bp DNA template is amplified by *pfu* DNA polymerase with flanking primers. The amplified template is purified and quantified for the DNA binding assay. To make specific-blocking DNA, mutant p53 consensus DNA (AGGCAAGgCaGGCAAGgCa) (SEQ. I.D. #6) is cloned into the same restriction sites for the wild type sequences as described above with the exception that XhoI and BamHI site are abolished. The 5 blocking primer (AACGCCAGGGTTTTCCCA) (SEQ. I.D. #7) and the 3 blocking primer (TATCGATACCGTCGACCT) (SEQ. I.D. #8) that exclude the template primer sequences may be used for making blocking DNA. The blocking DNA is desirably identical to the specific template DNA except for mutated binding sites and lack of primer sequences for the template DNA so that the blocking DNA can neither be recognized by p53 and ER proteins nor be amplified by PCR with template primers but can provide competition for non-specific binding of proteins to reduce the likelihood of non-specific binding to specific template DNA.

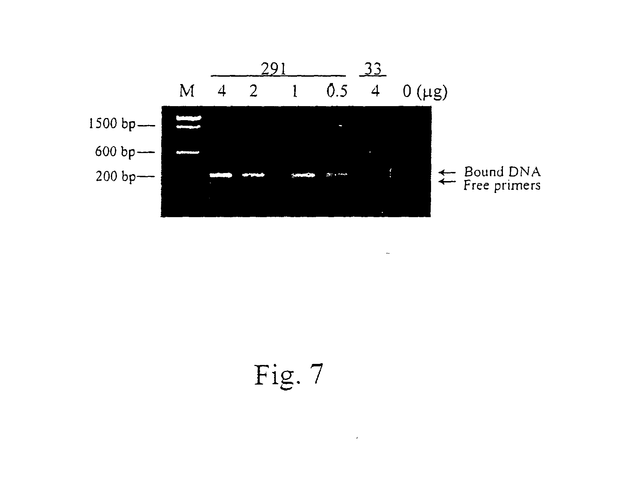

DNA binding reactions can be performed in DNA binding solution containing high salt extracts of tissues or cells to be tested, 0.1 pmol of template DNA, 10 pmol of blocking DNA, and 10 ng of specific antibody to capture the corresponding DNA-protein complex. After restriction digestions eliminate unbound and non-specifically bound DNA templates, the reaction is transferred to a fresh microtiter plate coated with secondary antibody (or protein-A or streptavidin if the primary antibody is biotinylated). Transfer is necessary to eliminate non-specific DNA binding to solid matrix. Non-specific DNA binding can be further reduced by using v-shaped microwells which offer a smaller surface area for non-specific binding. The specifically bound DNA templates are eluted and subjected to PCR amplification. The amplified DNA is measured by agarose gel electrophoresis and ethidium bromide staining (Fig. 7) or by the following detection systems for better quantification. Ethidium bromide staining detects minimally 10 ng DNA, equivalent to 5×10¹⁰ molecules of 210 bp DNA. it is not feasible to amplify a few DNA templates to the level detectable by ethidium bromide staining. In order to improve the sensitivity of the assay to the level capable of detecting DNA binding events in a few hundred cells, the enzyme-linked oligonucleotide sorbent assay (ELOSA) can be modified for this purpose. ELOSA is an ELISA-based technique that detects hapten-labeled oligonucleotide instead of antigen. Generally, the sensitivity of ELOSA is 0.1-1 fmol (10⁷-10⁸ molecules), far more sensitive than ethidium bromide staining. In addition, it has a broader linear range for quantification than ethidium bromide staining. Bound template DNA may be amplified with 5' and 3' template primers labeled with biotin and fluorescein, respectively. As a result, the amplified DNA will be labeled with fluorescein at one end and biotin at the other end so that DNA can be captured by streptavidin on a microplate and detected by anti-fluorescein-alkaline phosphatase conjugate with chromogenic para-nitrophenylphosphate (pNPP). The sensitivity of the assay can be measured by means of serial dilutions of cell lysate of know cell number and protein concentration.

Specifically Figure 7 shows analysis of DNA binding activity of p53 by DNA affinity PCR. Indicated amounts of lysate from non-transformed epidermal cell 291 (w.t. p53) and human cervical carcinoma cell C-33 (mt. p53) were subjected to DNA affinity PCR with a 210 bp DNA duplex containing p53 consensus site and PAB421 in a microtiter plate coated with anti-mouse IgG. The bound DNA was amplified by PCR and visualized by ethidium bromide staining.

Alternatively, bound DNA can be measured by light PCR with molecular beacons as primers (Tayca S., and Kramer FR., (1996), "Molecular beacons: probes that fluoresce upon hybridization", Nature Biotechnology *14*, 303-308). Molecular beacons are single-stranded oligonucleotides that contain a fluorescent dye on the 5' end and a quencher molecule on the 3' end. In the absence of DNA template, the molecular beacon forms a hairpin loop, placing the fluorophore adjacent to the quencher which results in no fluorescence. In the presence of DNA template, the molecular beacon binds to the complementary sequence of the template, separating the fluorophore from the quencher. This fluorescent signal is proportional to the amount of amplified DNA in the reaction and can be detected during PCR with a spectrofluorometric thermal cycler (Wittwer C.T., Herrmann M.G., Moss A.A., and Rasmussen R.P., (134), "Continuous fluorescence monitoring of rapid cycle DNA amplification", Biotechniques *22*, 130-131). The signals can be plotted at each amplification cycle. The initial template number can be calculated based on the number of cycles required for the level of signal detectable by the spectrofluorometric thermal cycler. The more bound DNA for the initial amplification, the fewer amplification cycles needed to reach the threshold of fluorescent detection.

By DNA affinity PCR, it is possible to provide positive and negative readouts of p53 function capable of extending the molecular profiling of patient tumors beyond IHC alone. However, the ability to calculate a functional index provides additional advantages for avoiding false positives and false negatives and for further categorizing biologically meaningful activities in individual tumors. Another embodiment of the present invention provides a highly specific and sensitive detection system for quantification of binding proteins such as p53 and ER. Immuno-PCR has been developed for the detection of trace amounts of antigen (Sano T., Smith C.L., and Cantor C.R., (1992), "Immuno-PCR: very sensitive antigen detection by means of specific antibody-DNA conjugates", Science *258*, 120-122). This is an extremely sensitive technique that combines the specificity of antigen-antibody interaction and the sensitivity of PCR. In immuno-PCR, antigen to be detected is absorbed on microplates and recognized by the specific antibody. The detection of bound primary antibody can be achieved either by a bispecific fusion protein of protein A and streptavidin or by biotinylated secondary antibody and biotinylated DNA in conjunction with streptavidin (Sano T. and Cantor C.R. (1991), "A streptavidin-protein A chimera that allows one-step production of a variety of specific antibody conjugates", Bio/Technology *9*, 1378-1381). The biotinylated DNA molecule can be amplified by PCR. However, immuno-PCR has not been developed as a reliable tool for general practice. Since the specificity of immuno-PCR is only dependent on antigen-antibody interactions, unavoidable non-specific interactions greatly limits the power of PCR for sensitivity. In addition, the absorbing efficiency of antigen to the solid matrix varies in different samples due to competing binding sites with other molecules. These drawbacks greatly restrict the application of immuno-PCR in general practice. To improve the specificity of immuno-PCR, the present invention offers specific interactions in addition to the antigen-antibody interaction. The additional, specific interaction is achieved using specific ligand labeled with biotin or fusion proteins containing a streptavidin moiety and the associated protein. By designing different streptavidin fusion proteins based on the biochemical properties of the protein of interest, this technique can be applied for general detection of extremely low protein concentrations, with enhanced specificity and functionality.

### Example 1: p53 Quantification

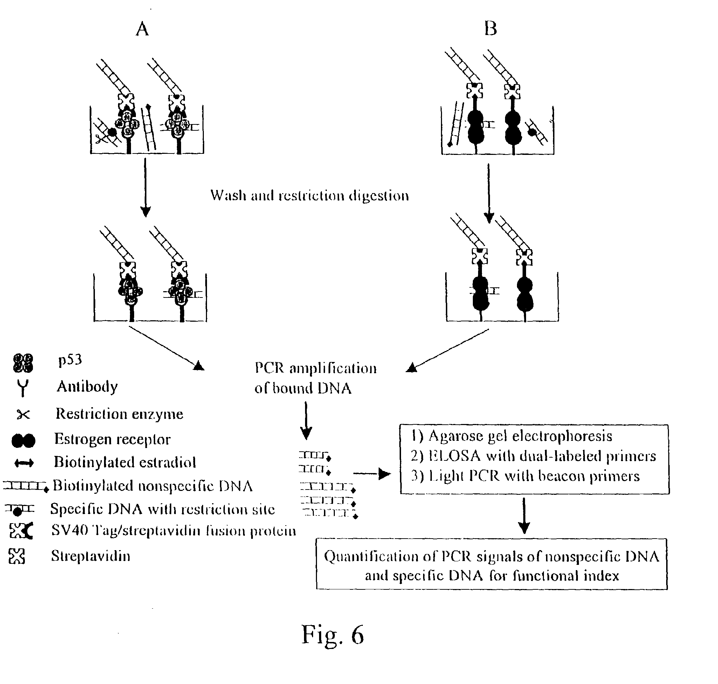

p53 protein is a transcription factor composed of several distinct domains for transactivation, DNA binding, oligomerization, and DNA binding regulation. A number of cellular proteins have been identified as being associated with p53 protein. These associated proteins are potential candidates for constructing a streptavidin fusion protein. As the first p53 associated protein identified, SV40 large T antigen is selected for making the initial fusion protein for p53 detection based on a high affinity interaction. This interaction results from multiple contacts between p53 and SV40 large T antigen over a broad surface area. SV40 LT antigen binds to two separate regions of p53, including residues 123-215 and 232-285, and encompasses the entire central DNA binding region of p53 (Jenkins J.R., Chumakov P., Addison D., Sturzbecher H.W., and Wade-Evans A., (1988), "Two distinct regions of the murine p53 primary amino acid sequence are implicated in stable complex formation with simian virus 40 T antigen", Journal of Virology, *62*, 3903-3906; Tan T.H., Wallis J., and Levine A.J., (1986), "Identification of the p53 protein domain involved in formation of the simian virus 40 large T-antigen-p53 protein complex", Journal of Virology, *59*, 574-583). The strategy for this SV40 LT antigen-based p53 detection assay is schematically illustrated in Figure 6.

A fusion protein of streptavidin and SV40 Tag can be constructed by cloning a DNA fragment, encoding a polypeptide corresponding to residues 84-708 of T antigen (Li B. and Fields S., (1993), "Identification of mutations in p53 that affect its binding to SV40 large T antigen by using the yeast two-hybrid system", FASEB Journal 7, 957-963), into the pTSA-18F plasmid (Sano T. and Cantor C.R., (1991), "A streptavidin-protein A chimera that allows one-step production of a variety of specific antibody conjugates", Bio/Technology *9*, 1378-1381), a vector in which the expression of streptavidin is under the control of bacteriophage T7 promoter. The resultant vector encodes a fusion protein of T antigen followed by the C-terminus of streptavidin with 10 additional amino acid residues between the two moieties. The streptavidin fusion protein is purified by affinity chromatography with 2-iminobiotinagarose. The bi-specificity of the fusion protein is determined by immunoprecipitation of p53 with biotinylated protein A Sepharose CL-4B beads. ³⁵S-methionine labeled p53 can be *in vitro* translated as described by Wu, et al. (Wu Y., Liu Y., Lee L., Miner Z., and Kulesz-Martin M., (1994), "Wild-type alternatively spliced p53: binding to DNA and interaction with the major p53 protein *in vitro* and in cells", EMBO Journal *13*, 4823-4830) and incubated with purified T antigen-streptavidin fusion protein followed with biotinylated protein A-Sepharose CL-4B beads.

A detection reaction is performed in a microtiter plate on which p53 specific antibody is coated to capture total p53 protein. The captured p53 protein can be recognized by the fusion protein of SV40 Tag and streptavidin, which is capable of capturing biotinylated DNA template. The captured DNA fragment is eluted and amplified by PCR. Agarose gel electrophoresis and ethidium bromide staining or ELOSA, or light PCR, may be used to measure the amplified DNA. The quantification of amplified DNA may be used, e.g. as a denominator, for calculating a functional binding index of p53 protein in combination with the result of p53 DNA binding by DNA affinity PCR.

Alternatively, the quantification of p53 protein and active p53 protein can be carried out in a single reaction as shown in Figure 6A. Active and total p53 proteins can be differentially recognized by p53 specific DNA template and SV40 T antigen-streptavidin fusion protein in the presence of biotinylated non-specific DNA template. To avoid inhibitory effect of SV40 T antigen on p53 DNA binding, specific DNA template should react first with p53 protein then biotinylated non-specific DNA is added with SV40 T antigen-streptavidin fusion protein. The captured DNA fragments are eluted and amplified by PCR with corresponding primers. The DNA fragments amplified from specific and non-specific DNA can be distinguished by the size in agarose gel electrophoresis followed by ethidium bromide staining. Alternatively, the amplified DNA fragments can be detected by ELOSA with one primer labeled with biotin and the other with fluorescein or digoxigenin so that specific and non-specific DNA can be differentially detected by horseradish peroxidase conjugate for fluorescein and alkaline phosphatase conjugate for digoxigenin. More preferably, they can be detected by real time PCR with primers labeled Cy5 (emission 675nm) or Cy3 (emission 567nm). The quantification of amplified DNA will be used as a denominator for calculating functional index of p53. The functional index of p53 is determined by dividing signal intensity of specific DNA with signal intensity of non-specific DNA. Functional index of p53=[signal intensity of specific DNA (active p53 protein)]÷[signal intensity of non-specific DNA (total p53 protein)].

### Example 2: Estrogen Receptor (ER) Quantification

Micro-assay for ER quantification, in accordance with the invention, takes advantage of the estrogen binding property of ER. Estradiol is the ligand of ER that binds to the carboxy-domain of ER. Biotinylated estradiol complexes with biotinylated DNA template and streptavidin because it is a tetrameric molecule capable of binding four biotin moieties (Fig. 5C). Estrogen receptor in the cell lysate can be immobilized on a solid matrix coated with ER specific antibody. In this assay, monoclonal anti-ER antibody that recognizes the ER amino-terminal domain is used since it should not cause steric hindrance to estradiol binding at the carboxy-terminal domain of ER. The captured ER may interact with biotinylated estradiol to which biotinylated DNA template may be captured in the presence of tetrameric avidin. The bound DNA can be amplified by PCR and detected by agarose gel electrophoresis and ethidium bromide staining or ELOSA, or light PCR. Quantification of ER through its natural ligand also has the advantage of reflecting the ligand binding property of ER. Defects in ligand binding represent a fair fraction of ER abnormalities in breast cancer. Since the specificity of the assay is defined by both antibody and estrogen ligand, it is more specific than ELISA. The integration of estradiol in the assay adds a functional aspect to the detection of ER since a defect in estrogen binding is one of the mechanisms of ER inactivation in breast cancer.

The primary antibody can be coated in a microtiter plate with 5% nonfat mild powder-blocking agent to block non-specific sites on the microtiter plate (instead of using bovine serum albumin (BSA), since albumin is an estrogen carrier in blood circulation, with a Kd value of 10⁻⁶M). The reaction can be performed in solution containing lysates of tissues or cells to be tested and a complex of biotinylated estradiol, neutravidin, and biotinylated DNA template (Tiefenauer L.X. and Andres R.Y., (1990), "Biotinyl-estradiol derivatives in enzyme immunoassays: structural requirements for optimal antibody binding", Journal of Steroid Biochemistry, *35*, 633-639). The advantage of using neutravidin is that it has an isoelectric point closer to neutrality than avidin or streptavidin. This minimizes electrostatic interactions that can contribute to non-specific binding. Captured ER may interact with biotinylated estradiol to which biotinylated DNA template may be captured in the presence of tetrameric avidin. The bound DNA can be amplified by PCR and detected by agarose gel electrophoresis and ethidium bromide staining or ELOSA, or light PCR. Quantification of ER through its natural ligand also has the advantage of reflecting the ligand binding property of ER. Defects in Ligand binding represent a fair fraction of ER abnormalities in breast cancer. Since the specificity of the assay is defined by specific antibody and estrogen ligand, it is more specific than ELISA. The integration of estradiol in the assay adds a functional aspect to the detection of ER since a defect in estrogen binding is one of the mechanisms of ER inactivation in breast cancer. Alternatively, active and total ER proteins can be differentially recognized by ER specific DNA template and biotinylated estradiol in the presence of streptavidin and biotinylated non-specific DNA template in a single reaction (Figure 6B). The captured DNA fragments are eluted and amplified by PCR with corresponding primers. The DNA fragments amplified from specific and non-specific DNA can be distinguished by size in agarose gel electrophoresis followed by ethidium bromide staining. Alternatively, the amplified DNA fragments can be detected by ELOSA with one primer labeled with biotin and the other with fluorescein or digoxigenin so that specific and non-specific DNA can be differentially detected by horseradish peroxidase conjugate for fluorescein and alkaline phosphatase conjugate for digoxigenin. More preferably, they can be detected by real time PCR with primers labeled Cy5 (emission 675nm) or Cy3 (emission 567nm). The quantification of amplified DNA will be used as a denominator for calculating functional index of ER protein. The functional index of ER is determined by dividing signal intensity of specific DNA with signal intensity of non-specific DNA. Functional index of ER=[signal intensity of specific DNA (active ER protein)]÷[signal intensity of non-specific DNA (total ER protein)]. The quantification of amplified DNA can be used as a denominator for calculating functional index of ER in combination with the result of ER DNA binding by DNA affinity PCR.

Specifically Figure 6 shows a schematic illustration of quantitative DNA affinity assays. 6A illustrates quantification of p53 DNA binding by DNA affinity PCR. Total p53 protein in the lysate will be captured by p53 antibody immobilized on a solid matrix. The specific DNA of one size will bind to active p53 protein then a biotinylated non-specific DNA of a different size will be added to bind total p53 protein in the presence of SV 40 Tag-streptavidin fusion protein. Specific and non-specific DNA captured will be amplified by their corresponding primers. 6B illustrates quantification of ER DNA binding by DNA affinity PCR. Total ER protein in the lysate will be captured by ER antibody immobilized on a solid matrix. The specific DNA binds only to active ER protein whereas biotinylated non-specific DNA binds to total ER protein in the presence of biotinylated estradiol and streptavidin. Specific and non-specific DNA captured will be amplified by their corresponding primers. The amplified DNA products are detected and quantified, e.g. by 1) agarose gel electrophoresis (different sizes); 2) enzyme-linked oligonucleotide sorbent assay (ELOSA) with one primer labeled with biotin and the other with fluorescein or digoxigenin (different enzyme conjugates); or 3) PCR with oligonucleotide labeled with Cy5 or Cy3 (different emission wavelength) by spectrofluorometric thermal cycler. The quantity of PCR signal of specific DNA binding and non-specific DNA trapping is expressed as ratio for functional index.

Quantification of p53 and ER by modified immuno-PCR provides quantitative values for calculating their functional indices. However, certain p53 mutations within the Tag binding region of p53, including 143, 175, 248, and 273 hotspots, may not be detected by this Tag-based immuno-PCR. Since most of these mutants are not capable of DNA binding, the functional index will be presented as p53 null instead of p53 mutation. On one hand, since p53 null and p53 mutation are considered clinically to be the same, the results from each scenario may be equivalent and should be biologically significant. On the other hand, it is advantageous to differentiate positive DNA binding from wild type p53 protein and 273 mutant by this Tag-based p53 detection assay. Mutation within codon 273 is one of the most common p53 mutations, accounting for 13% of total p53 mutation. p53 protein with the 273 mutation is capable of mediating transactivation and active DNA binding. 273 mutants will be identified as those positive for DNA binding but negative for binding Tag. Since SV40 T antigen binds to p53 over almost the entire DNA binding domain in a conformation-dependent manner, the fusion protein of SV40 T antigen and streptavidin (described in this invention) provides a better means for analysis of p53 conformation than by ELISA or immunostaining of frozen tumor tissue section using conformation-dependent monoclonal antibody, which is only an epitope composed of a few amino acids. The assay of p53/SV40 T antigen alone has the potential to be developed into a simple and sensitive functional assay for p53. In the case of estradiol-based ER detection, certain ER proteins with alterations in the C-terminal domain important for ligand binding will not be detected. This property of the assay could be a major advantage in analysis of ER function since some ER variants with defects in ligand binding are capable of binding DNA. In addition to being a denominator of functional index for ER DNA binding, this estradiol-based immuno-PCR serves as a complement for ER functional assessment.

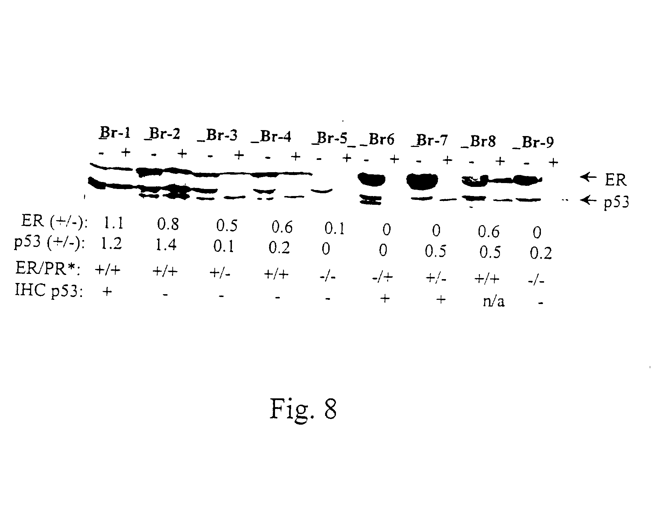

An objective of the present invention is to provide reliable prognostic markers for physicians and patients to make decisions about therapeutic regimens and to monitor clinical course during treatment based on molecular defects in individual cancer patients, e.g. breast cancer. Reliable prognostic markers and reliable predictors of a patient's response to particular therapies will provide information for more rationale selection of optimal therapy for the individual patient and for avoidance of unnecessary toxicity. Wild type p53 mediates cell cycle checkpoints, allowing the repair of damaged DNA or triggering apoptosis in cells with irreparable DNA damage. p53-mediated apoptosis is a common mechanism of action for various chemotherapeutic drugs. The group of patients with the poorest outcome in breast cancer, for example, includes those with IHC positive p53 and positive Her2/neu receptor, implying the importance of p53 status in breast cancer patients. Yet p53 testing, even by IHC is not applied in current practice for these patients and p53 mutation as a reliable prognostic marker in selection of effective treatments is still controversial. A functional assay should be more powerful in establishing predictive value of proteins. The DNA binding activity of p53 protein and its correlation with p53 mutation, as well as transactivation of downstream genes, have been studied extensively by EMSA. These studies, however, generally involve cell culture samples, technical conditions, and methods less amenable to direct application to analysis of clinical tissue specimens. By contrast, the present invention permits testing in a clinical setting. In order to extend the DNA binding assay to measuring p53 sequence-specific DNA binding, DAI can be used on tissue lysates prepared from clinical specimens (Fig. 8). Significant DNA binding activity of p53 was detected in clinical specimens from breast cancer patients with functional indices ranging from 1.3 to 0.26. To compare with conventional approaches for p53 mutation, these tumor specimens were examined by IHC with DO-1, the same antibody used in DAI. The IHC staining pattern was heterogeneous among the tumors. While some exhibited nuclear staining, others showed diffuse staining in the cytoplasm. Positive p53 staining was seen in 10 of the 20 specimens (50%). Agreement of p53 status between DAI and IHC results was reached in only 60% of the specimens (Table 1). Significant DNA binding was observed in 2 cases with IHC positive p53. In 5 other cases, IHC failed to detect nonfunctional p53 proteins that were detected by immunoblotting and DAI. The discordance between DAI and IHC suggests that 1) not all IHC detectable p53 represent p53 mutations, and 2) not all the inactive p53 proteins are IHC detectable. In the case of estrogen receptor, not all ER proteins detected by IHC are capable of binding to DNA. Significant DNA binding was undetectable in nearly a quarter of ER positive tumor specimens, which, strikingly, is parallel to the proportion of ER positive patients who are non-responsive to hormonal therapy. Figure 8 illustrates the above in more detail. Specifically, Figure 8 shows DNA binding activities of p53 and ER in human breast cancer by DAI. Total tissue lysates were prepared from frozen human breast cancer specimens of 100 to 200 mg wet weight. A 200 µg aliquot of tissue lysate from each specimen was subjected to DAI (+) with a biotinylated DNA cocktail of p53 and ER probes. Forty µg of corresponding tissue lysates were loaded next to the DAI samples as relative total protein (-). The samples were fractionated through 10% SDS-gel and blotted with antibodies for ER and p53. The functional index is calculated by dividing the signal of DAI (+) by total protein (-). Paraffin sections from corresponding breast tumor specimens were stained with DO-1 polyclonal antibody for p53 protein. *The IHC results of ER and progesterone (PR) were provided by the RPCI Translational Research Tissue Support program. The present invention shows that DNA binding function can be measured and used to characterize further subsets of patient tumors. Permitting a therapeutic regimen to be tailored for individual patients according to their DNA binding profile.

**Table 1.**

| Comparison of DAI with IHC for p53 and ER status in breast cancer patients | | | | | | |
|---|---|---|---|---|---|---|
| # | Date | Diagnosis | estrogen receptor | | p53 | |
| | | | IHC/PR* | Functional index | IHC | Functional index |
| B1 | 7/29/97 | Invasive Ductal Ca | -/+ | 0.01 | + | 0.01 |
| B2 | 9/30/97 | In-situ Ductal Ca | +/- | 0.00 | + | 0.44 |
| B3 | 8/5/97 | Invasive Lobular Ca | +/+ | 0.64 | N/A | 0.48 |
| B4 | 5/7/99 | Poorly Diff. Aden. Ca | -/- | 0.01 | - | 0.15 |
| B5 | 2/3/98 | Invasive Ductal Ca | +/+ | 1.12 | + | 1.18 |
| B6 | 5/29/98 | In-situ Lobular Ca | +/+ | 0.80 | - | 1.36 |
| B7 | 9/8/98 | Invasive Ductal CA | +/- | 0.51 | + | 0.09 |
| B8 | 10/9/98 | poorly Diff. Ductal Ca | +/+ | 0.62 | - | 0.18 |
| B9 | 11/27/98 | In-situ Ductal Ca | -/- | 0.05 | - | 0.02 |
| B10 | 6/30/98 | Invasive Ductal Ca | +/+ | 0.00 | - | 0.03 |
| B11 | 2/1/94 | Invasive Ductal Ca | +/+ | 0.37 | - | 0.07 |
| B12 | 2/23/94 | Invasive Ductal Ca | -/- | 0.00 | - | 0.26 |
| B13 | 9/13/94 | Invasive Ductal CA | +/+ | 0.12 | + | 0.01 |
| B14 | 6/7/94 | Invasive Ductal CA | -/- | 0.07 | + | 0.02 |
| B15 | 7/5/94 | Invasive Ductal CA | N/A | 0.01 | + | 0.02 |
| B16 | 1/12/94 | Invasive Ductal Ca | N/A | 0.10 | + | 0.03 |
| B17 | 1/12/94 | In-situ Ductal CA | +/+ | 0.02 | + | 0.03 |
| B18 | 3/30/94 | Invasive Ductal CA | +/+ | 0.69 | + | 0.07 |
| B19 | 4/6/94 | Invasive Ductal CA | +/+ | 0.91 | - | 0.29 |
| B20 | 5/25/94 | Invasive Ductal CA | -/- | 0.00 | - | 0.00 |
| B21 | 9/6/94 | Invasive Ductal CA | N/A | 0.23 | - | 0.05 |
| Positive % | | | 66% | 40% | 50% | 30% |
| % correlation with IHC | | | | 77% | | 60% |

## Claims

1. A method for quantifying a DNA binding protein interaction with DNA in a biological sample, in the absence of the use of a radioisotope, and at a sensitivity greater than the electrophoretic mobility shift assay (EMSA), said method being **characterized in that** it comprises:
a) treating the sample to obtain a liquid to be tested for the DNA binding protein,
b) incubating the liquid containing the DNA binding protein with a DNA, containing a binding site to which DNA binding protein can specifically bind so that DNA binding protein specifically binds to the DNA,
c) separating the DNA with bound DNA binding protein from other DNA and proteins in the liquid, and
d) detecting the separated DNA binding protein, in the absence of the use of a radioisotope, and at a sensitivity greater than EMSA, using a test that yields a first numerical result proportional to the quantity of separated DNA binding protein, said test being selected from the group consisting of immunological quantification of DNA binding protein separated with the bound DNA and PCR amplification of DNA separated with the DNA binding protein followed by quantification of the PCR amplified DNA.

2. The method of claim 1, **characterized in that** total protein in the sample, having a specific immunoreactive site in common with a specific immunoreactive site of the separated DNA binding protein, is detected using a test that yields a second numerical result proportional to the quantity of said total protein and comparing the first numerical result with the second numerical result to obtain an index reflecting relative proportion of bound and unbound protein in said total protein.

3. The method of claim 1 **characterized in that** the DNA, containing a binding site to which DNA binding protein can specifically bind, is treated with a material to provide a site that permits the treated DNA to be separated from other DNA in the liquid without interfering with the binding sequence, the liquid containing the DNA binding protein is incubated with the treated DNA so that DNA binding protein specifically binds to the DNA, and the treated DNA with bound DNA binding protein is separated from other DNA in the liquid using the provided separation site.

4. The method of claim 1 **characterized in that** DNA binding protein interaction with DNA is detected by a method comprising:
a) immobilization of DNA binding protein in the biological sample on a solid matrix,
b) incubating DNA binding protein with DNA template containing a site to which the DNA binding protein can specifically bind,
c) reducing non-specific binding by including DNA duplex that is non-specific to said DNA binding protein,
d) eliminating non-specific DNA by restriction digestion,
e) amplification of specifically bound DNA by polymerase chain reaction, and
f) quantification of amplified DNA.

5. The method of claim 3 **characterized in that** the DNA is biotinylated and is removed from the liquid by reaction with streptavidin attached to a substrate.

6. The method of claim 5 **characterized in that** the binding protein is freed from the DNA after removal of the DNA and is subjected to SDS-gel fractionation followed by quantification by immunoblotting.

7. The method of claim 6 **characterized in that** the binding protein is p53 which is immunoblotted using p53 antibody.

8. The method of claim 3 **characterized in that** the DNA is treated by providing a restriction site in all DNA in the liquid, which restriction site can be protected, by bound binding protein, from destruction by a restriction enzyme specific for the restriction site and DNA bound to binding protein is separated from other DNA in the liquid by exposing DNA in the liquid to the restriction enzyme thus destroying non-bound DNA.

9. The method of claim 8 **characterized in that** the bound DNA, after being separated from other DNA in the liquid, is subjected to polymerase chain reaction (PCR) amplification and the amplified DNA is quantified.

10. The method of claim 9, **characterized in that** the amplified DNA is quantified by a detection method selected from the group consisting of agarose gel electrophoresis with DNA staining, enzyme linked oligonucleotide sorbent assay (ELOSA) or PCR with beacon oligonucleotide as primers by spectrofluorometric thermocycler.

11. The method of claim 2 **characterized in that** the index is used to determine the course of treatment for a patient.

12. The method of claim 2 **characterized in that** the index is used to determine prognosis for a patient.

13. The method of claim 3, or 5-10 **characterized in that** total protein in the sample, having a specific immunoreactive site in common with a specific immunoreactive site of the separated DNA binding protein, is detected using a test that yields a second numerical result proportional to the quantity of said total protein and comparing the first numerical result with the second numerical result to obtain an index reflecting the amount of separated bound DNA binding protein compared with said total protein.

14. The method of claim 2 **characterized in that** said total protein in the sample is quantified by subjecting a sample of the liquid containing the total protein to SDS-gel fractionation followed by quantification by immunoblotting in parallel with immunoblotting to determine binding protein, using a same antibody reactive with the immunoreactive site and comparing the quantity of said total protein with the quantity of binding protein to obtain said index.

15. The method of claim 2 **characterized in that** the index is used to screen for activity of therapies and agents that alter activity of DNA binding protein favorable to treatment of disease.

16. The method of claim 1 **characterized in that** the biological sample comprises a cellular lysate.

17. A method for quantifying DNA binding of a DNA binding protein in a biological sample as claimed in claim 1 and comparing it with total protein in the sample having at least one specific immunoreactive site in common with the DNA binding protein, **characterized in that** said method comprises:
a) treating the sample to obtain a liquid containing the total protein including the DNA binding protein,
b) generating DNA templates, containing a site to which the DNA binding protein can specifically bind,
c) incubating the liquid containing the DNA binding protein with the DNA template so that DNA binding protein specifically binds to the DNA to form a complex,
d) removing the DNA template with bound DNA binding protein from the liquid,
e) quantifying DNA bound protein and said total protein, and
f) comparing bound protein with total protein to obtain an index reflecting the relative proportions of bound and unbound protein in said total protein.

18. The method of claim 17 **characterized in that** the index is used to screen for activity of therapies and agents that alter activity of DNA binding protein favorable to treatment of disease.

19. The method of claim 18 **characterized in that** the disease is cancer.

20. The method of claim 17 for detecting active DNA binding protein in a biological sample **characterized in that** the method comprises: biotinylating a duplex DNA comprising a specific binding site for active DNA binding protein to create the DNA template, removing the DNA template with bound DNA binding protein from the liquid using streptavidin and quantifying DNA bound protein relative to total protein using side by side immunoblotting with the same antibody.

21. The method of claim 20 **characterized in that** it is used for identification of sequence specific DNA binding proteins.

22. The method of claim 20 **characterized in that** it is used for screening compounds, proteins, and reagents that target DNA-protein interactions.

23. The method of claim 20 **characterized in that** it is used for simultaneously detecting multiple DNA binding proteins having different molecular weights.

24. The method of claim 20 **characterized in that** the biotinylated DNA has unique structures based at least partly upon characteristics other than sequence.

25. The method of claim 24 **characterized in that** the unique structure is a Holliday structure.

26. The method of claim 24 **characterized in that** the unique structure is a DNA mismatch structure.

27. The method of claim 20 **characterized in that** the DNA-protein complex is further **characterized by** microsequencing of gel fractionated polypeptide.

28. The method of claim 20 **characterized in that** the DNA-protein complex is further **characterized by** Surface Enhanced Laser Desorption-Ionization Mass Spectroscopy.

29. The method of claim 17 **characterized in that** the sample is a cell lysate and the separated bound DNA is amplified by PCR wherein an antibody specific for the DNA binding protein is used to separate the DNA-protein complex from the sample to a solid matrix.

30. The method of claim 29 **characterized in that** the solid matrix is a microtiter plate.

31. The method of claim 29 **characterized in that** a DNA that is non-specific to said binding protein is added to the sample to reduce non-specific binding of proteins other than the DNA binding protein to the DNA template.

32. The method of claim 31 **characterized in that** the DNA that is non-specific to said binding protein is a non-specific DNA duplex identical to the DNA template except that the DNA binding site for the binding protein is mutated.

33. The method of claim 29 **characterized in that** the DNA template comprises a duplex DNA containing sequences that flank the binding site that are complementary to corresponding PCR primers for amplification.

34. The method of claim 29 **characterized in that** the DNA template comprises a duplex DNA having restriction endonuclease sites integrated in spacer and flanking regions of the binding site which can be protected from digestion by restriction endonuclease by protein bound to the binding site while unbound DNA remains subject to digestion by restriction endonuclease.

35. The method of claim 2 **characterized in that** the total protein is total p53 protein that is detected by a method comprising:
a) immobilizing total p53 protein in the biological sample on a solid matrix,
b) binding of a bifunctional fusion molecule that binds both p53 protein and biotin to the immobilized p53 protein,
c) binding biotinylated DNA, not specific for p53 protein binding, to the bound fusion protein,
d) amplifying the bound biotinylated DNA by polymerase chain reaction, and
c) quantifying the amplified DNA.

36. The method of claim 35 **characterized in that** the capture agent is streptavidin.

37. The method of claim 35 **characterized in that** the binding protein is p53 and the capture agent is SV40 Tag and the first numerical result is proportional to the quantity of p53 binding protein in the sample.

38. The method of claim 37 **characterized in that** it uses a bifunctional fusion protein of SV40 Tag and streptavidin that is specific to wild type p53 and biotin.

39. The method of claim 35 **characterized in that** it uses a biotinylated DNA duplex having DNA sequences that are complementary to corresponding PCR primers for amplification.

40. The method of claim 37 **characterized in that** total p53 type protein in the sample, having a specific immunoreactive site in common with a specific immunoreactive site of the separated DNA p53 binding protein, is detected using a test that yields a second numerical result proportional to the quantity of said total p53 type protein and comparing the first numerical result with the second numerical result to obtain an index reflecting relative proportion of bound and unbound p53 type protein in said total protein.

41. The method of claim 2 **characterized in that** the total protein is total estrogen receptor protein that is detected by a method comprising:
a) immobilization of said estrogen receptor protein in the biological sample on a solid matrix,
b) binding of biotinylated ligand estradiol to the immobilized estrogen receptor,
c) binding streptavidin to the bound biotinylated ligand estradiol,
d) binding biotinylated DNA, not specific for estrogen receptor binding, to the bound streptavidin,
e) amplification of bound biotinylated DNA by PCR, and
f) quantification of amplified DNA.

42. The method of claim 41 **characterized in that** the biotinylated DNA is a biotinylated DNA duplex having DNA sequences that are complementary to a corresponding PCR primer for amplification.

43. The method of claim 41 **characterized in that** the quantified amplified DNA is used as the second numerical result for calculation of the index reflecting relative proportion of bound and unbound estrogen receptor protein.

44. The method of claim 2 **characterized in that** the sample comprises a cellular lysate.

45. The method of claim 2 **characterized in that** the sample comprises a tumor cell lysate.

46. The method of claim 2 **characterized in that** the DNA binding protein is a nuclear protein.

47. The method of claim 1, 2 or 17 **characterized in that** a plurality of different DNA binding proteins having different molecular weights are simultaneously quantified using a plurality of DNAs, which contain binding sequences to which the different DNA binding proteins can specifically bind, wherein the DNAs containing the specific binding sequences are all treated with a material to provide a site that permits the treated DNAs to be separated from other DNA in the liquid without interfering with the binding sequences.
